# EUROPEAN PATENT SPECIFICATION

(11) **EP 0 447 582 B1**
(45) Date of publication and mention of the grant of the patent: **12.06.1996**
(21) Application number: 90105256.3
(22) Date of filing: 20.03.1990
(51) Int. Cl.: A01N 63/00

(54) **Method and composition for controlling color in a peroxide-Peroxidase disinfecting system**
Verfahren und Zusammensetzung zur Kontrolle der Farbe in einem desinfizierenden Peroxyd-Peroxydase-System
Procédé et composition pour contrôler la couleur dans un système de désinfection péroxyde-Péroxydase

(43) Date of publication of application: 25.09.1991
(73) Proprietor: Symbollon Corporation, Sudbury, MA 01776 (US)
(72) Inventor: Kessler, Jack, Dr., Southborough MA 01772 (US)
(74) Representative: Kinzebach, Werner, Dr.

(56) References cited:
- EP-A- 0 175 801
- EP-A- 0 307 376

## Description

A peroxide cold sterilization system which uses the enzyme peroxidase to catalyze the transfer of electrons from donor molecules to peroxide molecules in an aqueous solution is taught in U.S. Patent Nos. 4,476,231, and 4,473,550, and 4,588,586. The preferred donor molecule is a salt of iodide, preferably sodium or potassium iodide. A by-product of the peroxidase catalyzed reduction of hydrogen peroxide using an iodide salt as a source of donor molecules may be the formation of colored species.

A variety of disinfecting applications prohibit the formation of colored species. Said colored species present objectionable organoleptic properties when the disinfection occurs on humans or animals. These colored species may unacceptably alter the product to be disinfected in solution such as a contact lens or denture. Also there may be a threshold level of color which is objectionable in any application where said colored species can be visually identified during said enzymatically catalyzed disinfecting reaction.

EP-A-307 376 discloses a disinfection composition which comprises an iodide, lactoperoxidase and/or horseradish peroxidase and a hydrogen peroxide donor. The composition has a low iodide content, i.e. at least 10 ppm.

The term disinfection, as used in the present invention, means killing or rendering non-viable microscopic organisms found in nature. These organisms include, but are not limited to, Gram negative bacteria, Gram positive bacteria, fungi, and spores.

The peroxide sterilization system is dependent upon the relative concentration of peroxidase, peroxide and iodide in solution. For any given application, it is often necessary to utilize relatively high concentrations of iodide. This invariably increases the problem of the formation of colored species during the course of the enzyme-catalyzed reaction. It has been discovered that it is possible to control the formation of color when iodide is used as the donor molecule. The amount of color formed, once the disinfecting peroxidase-based chemistry of this application is initiated, increases as the iodide concentration increases. The rate and effectiveness of the disinfecting peroxidase-based chemistry of this application is usually positively correlated with the iodide concentration. Therefore, it is desirable to be able to reduce or eliminate the color contributed by the reaction between peroxidase, peroxide and iodide.

Color is defined as an increase in the spectral absorbence above a minimal threshold for a given application. In general, this increase can be defined as an increase in absorbence greater than 0.005 O.D. in a standard 1 cm cuvette as measured in a UV-VIS spectrophotometer falling in the wavelength range between 340 and 740 nanometers. That is, if the reaction generated by peroxidase, peroxide and iodide increases the absorbence of the reaction environment anywhere between 340 and 750 nanometers more than 0.005 O.D., then color is present. The absorbence maximum and shape of the absorbence spectrum generated by this system can vary depending upon the relative ratios and concentrations of the three components, and other variables, such as ionic strength and temperature.

It has also been discovered that it is possible to control the duration of color formation so as to have the disinfecting solution become colored upon dissolution of, for instance, a pill in a liquid carrier, and then have said solution turn colorless after the disinfecting cycle. Using an intrinsic color indicator to signal completion of a disinfection cycle confers a dramatic benefit to an end user of this technology in many applications.

The present invention contemplates the use of several methods to control the formation and duration of color. One method involves the addition of an agent to function as a decolorizer for the reaction between peroxidase, peroxide and iodide. Examples of suitable decolorizers include sorbic acid, boric acid, thiol group containing compounds and sodium chloride. The decolorizers can be added to the basic components of the peroxide system to form a color-controlled disinfecting peroxide sterilization composition, or can be introduced into the solution anytime during the reaction. The peroxide component may also be used as a decolorizer when present in a concentration above that required to fulfill the disinfecting application. For example, to disinfect a contact lens, the preferred peroxide concentration should be in a range of 0.0003 percent to 0.00003 percent. Additional peroxide can be added at a preferred range between 0.001 percent and 0.3 percent as a decolorizer, to the extent that it will not cause eye irritation to the lens wearer.

A second method which may be used in combination with the first mechanism to control the formation of color includes limiting the concentration of the iodide salt to a level equal to or below the level corresponding to the designated minimum acceptable color threshold level for the particular application, and adding other donor molecules to supplement the disinfecting formulation requirements for the application.

A third method to control color includes interrupting the reaction between the components, peroxidase, peroxide, and the iodide salt, upon reaching an acceptable color threshold for the particular application. The reaction may be interrupted in a variety of ways, including neutralizing the reaction by the addition of agents which reduce hydrogen peroxide, such as pyruvic acid, platinum, copper ions and catalase. Alternatively, an agent may be added to compete with the iodide salt in the reaction. The competing agent's activity in solution may be timed to increase as the acceptable minimum color threshold level is approached.

The above methods may be combined to supplement each other. For example, a decolorizer may be added to raise the minimum threshold level for unacceptable color formation in combination with the use of another donor molecule and/or a neutralizing agent.

The material used as a bactericide or disinfection agent, in accordance with the method of this invention, is a combination of a salt of iodide, a peroxide and a peroxidase, which can be stored in any fashion which precludes the initiation of the enzymatic reaction. That is, the components of this invention can be stored by combination of any two components without the presence of the third; this precludes the possibility of enzymatic action and subsequent depletion of substrates. Alternately, all the components of this invention can be stored as a powder or pill. The stored components of this invention are subsequently combined and thereby activated by admixture in a suitable carrier. When, decolorizers are added, they can also be predissolved in the carrier along with other components of the invention or they can be admixed as a component of a pill or powder.

Any solid entity which liberates hydrogen or methyl peroxide upon dissolution can serve as the source of peroxide. Alternately, one can dissolve the iodide salt and decolorizer(s) in a suitable carrier and make a pill containing peroxidase and a suitable source of peroxide.

The peroxidase of this invention is identified by the International Union of Biochemistry and the International Union of Pure and Applied Chemistry by the Enzyme Commission identification No. E.C. 1.11.17. Peroxidase can be obtained from a wide variety of sources. The least expensive and most robust peroxidase suitable for this application is horseradish peroxidase, although lactoperoxidase can also be used. Commercially obtained peroxidase comes lyophilized as a dry powder. Alternatively, peroxidase can be dissolved in a suitable carrier for future admixture with its substrates.

The donor molecule of this invention is iodide. Suitable sources of iodide for this invention include sodium iodide and potassium iodide, as well as other salts of iodide. Any compound which yields iodide ions upon dissolution in water, without yielding other deleterious effects to the activity of the system, is suitable for this application. The simple salts of iodide have the advantage of being inexpensive and they are very stable.

The oxidant of this invention is a compound which yields a peroxide upon dissolution in water or an appropriate carrier, although peroxide can be directly formulated into a liquid product.

The preferred oxidant of this invention is hydrogen peroxide. Alternatively, methyl peroxide can be formulated in the product. Suitable materials which can serve as precursors for hydrogen peroxide include metal peroxides, percarbonates, persulphates, perphosphates, peroxyesters, urea peroxide, peroxyacids, alkylperoxides, acylperoxides and perborates. Mixtures of two or more of these substances can also be used.

The decolorizers of this invention include a broad range of compounds. It has been found that certain soluble salts, such as sodium chloride or potassium chloride, or mixtures thereof, substantially reduces the color increase from the reaction between peroxidase, peroxide and iodide when the decolorizers are present in the 0.1 to 200 millimolar concentration range. It is to be understood that other alkali metal salts and alkali metal chlorides can often be substituted, in whole or in part, for the sodium or potassium chloride. Likewise, certain simple organic acids, such as sorbic acid, have been found to reduce or eliminate the color formed by the reaction of peroxides with iodide and peroxide. Likewise, thiol-containing compounds, such as dithiothreitol and mercaptoethanol have been found to reduce or eliminate the color formed by the reaction of peroxides with iodide and peroxide.

The decolorizers of this invention produce their action, while maintaining both peroxidase activity and the disinfecting effect of the chemistry. Sodium pyruvate has been used to inactivate the peroxide-based disinfection of contact lenses, but this type of inactivation is distinct from the control of an unwanted by-product, color, which this application describes. Color produced by the reaction of peroxidase, peroxide and iodide can be controlled by stopping the reaction after a defined period of time, by agents which inactivate peroxidase, consume peroxide or compete with iodide for the active site of peroxidase. However, this type of inactivation is different from that described in this application, since its effect is to terminate the disinfecting reaction. Sodium pyruvate, platinum or copper are typical examples of agents which consume peroxide. Hydrazine and methyl hydroperoxide are examples of agents which inactivate peroxidase. Fluoride is an example of an agent which will compete with iodide for the active site of peroxidase. Other donor molecules will also compete with iodide for the active site of peroxidase, but many of these molecules also yield color or are not suitable for a particular application. It is, however, possible to reduce the amount of color formed by substituting donor molecules such as tyrosine.

Temporal control of color formation during the enzymatically catalyzed reduction of peroxide by peroxidase in the presence of iodide can be effected by formulating the relative concentrations of iodide and hydrogen peroxide. The greater the iodide concentrations, all other things being equal, the more intense the color and the longer lasting the color. The concentration of hydrogen peroxide will effect the amount of color formed and shorten the time duration of color formation.

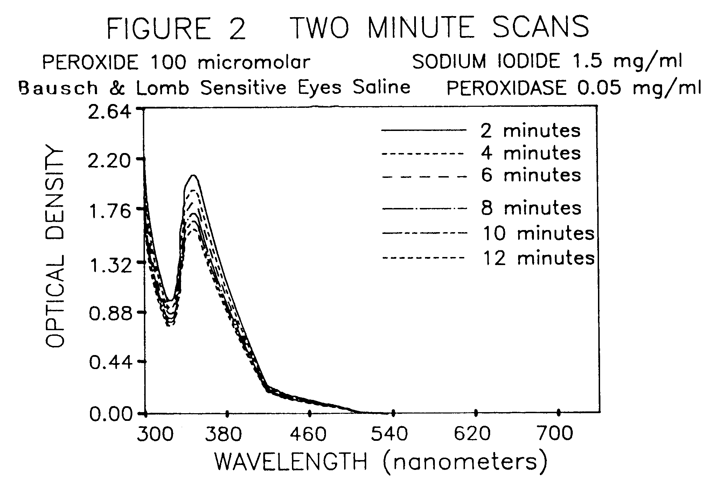

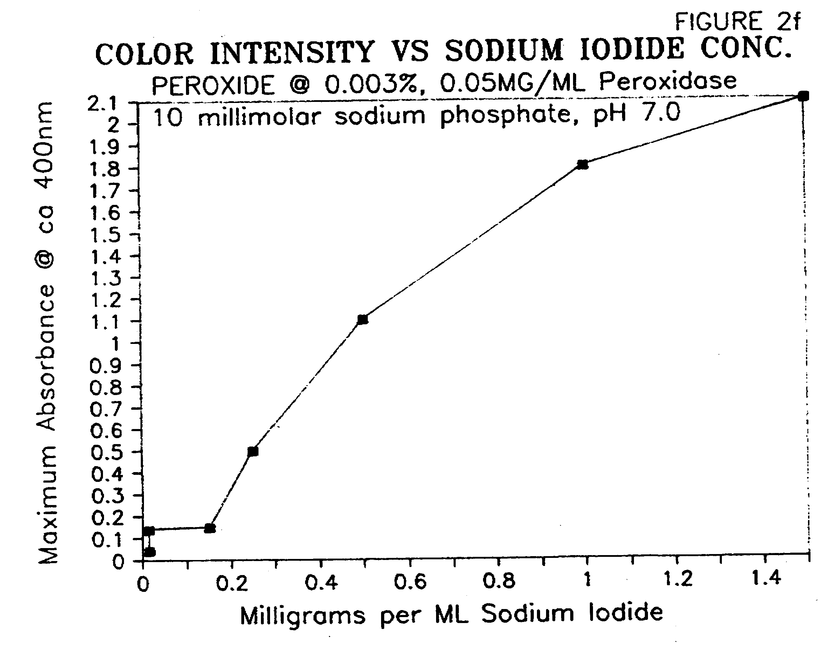

This invention deals with controlling color. The color intensity of a disinfecting reaction in the oral cavity must be controlled within an acceptable organoleptic range. In addition, the color present must not be so great as to stain or discolor the teeth. At component concentrations of 0.003 percent peroxide, 0.05 mg/ml peroxidase, 10 millimolar sodium phosphate (pH 7.0), a concentration of 0.1 mg/ml sodium iodide could be used in the oral cavity (Figure 2f) to maintain color at a suitable level.

### EXAMPLES

### EXAMPLE 1

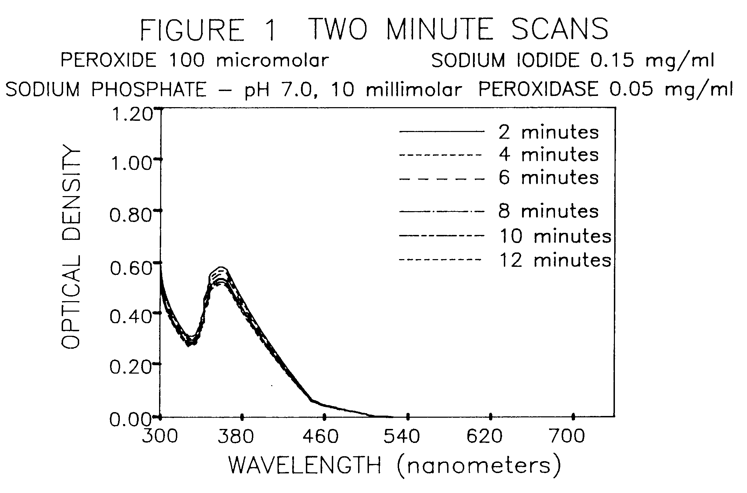

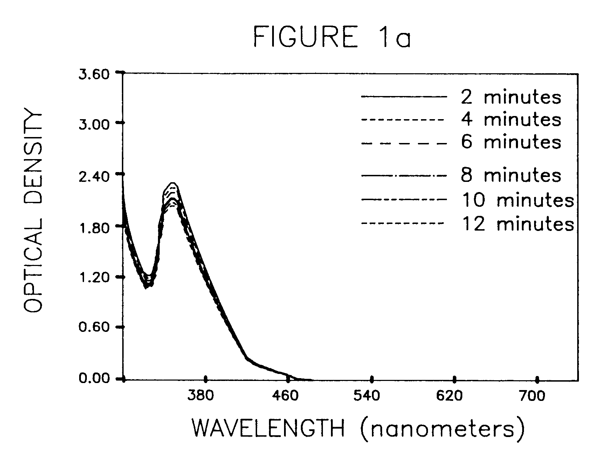

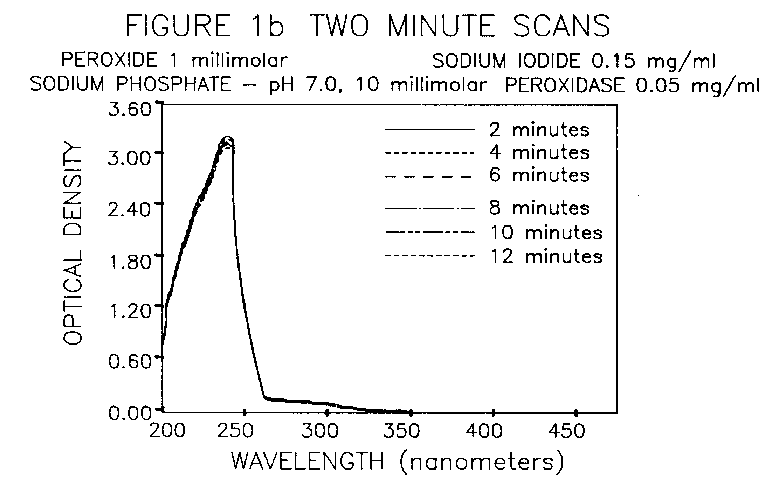

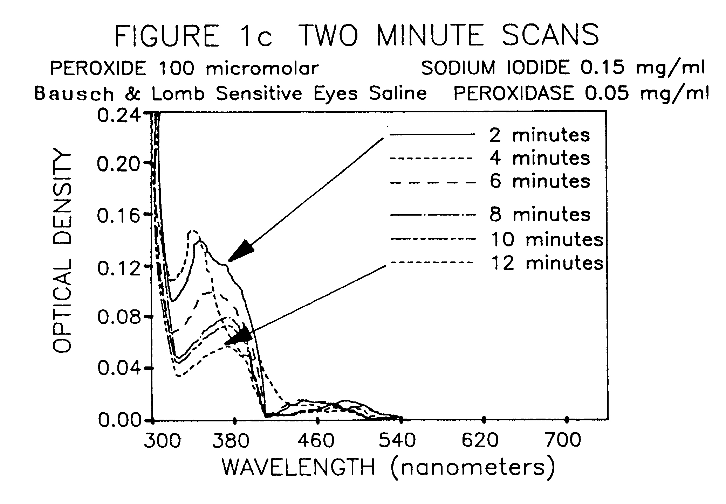

Figure 1 shows UV-VIS spectrophotometric scans taken at two-minute intervals on a Beckman 122 UV-VIS spectrophotometer. The volume was 3 ml containing the concentrations of components listed below.

| Concentration of Reagents for Figure 1 | |
|---|---|
| Component | Concentration |
| peroxidase | 0.05 mg/ml |
| peroxide | 100 micromolar |
| sodium iodide | 0.15 mg/ml |
| sodium phosphate-pH 7.0 | 10 millimolar |

Figure 1 shows a single absorbence peak of 0.550 optical density centered at 355 nm, which does not change meaningfully in intensity over a twelve-minute period. Figure 1a shows identical scans take at the following concentrations.

| Concentration of Reagents for Figure 1a | |
|---|---|
| Component | Concentration |
| peroxidase | 0.05 mg/ml |
| peroxide | 100 micromolar |
| sodium iodide | 1.5 mg/ml |
| sodium phosphate-pH 7.0 | 10 millimolar |

It is clear that the tenfold increase in sodium iodide concentration has increased the optical density to 2.25 O.D., an almost fivefold increase. Figure 1b shows the effect of increasing the effective concentration of peroxide tenfold, to 1 millimolar, at a concentration of sodium iodide of 0.15 mg/ml.

| Concentration of Reagents for Figure 1b | |
|---|---|
| Component | Concentration |
| peroxidase | 0.05 mg/ml |
| peroxide | 1 millilmolar |
| sodium iodide | 0.15 mg/ml |
| sodium phosphate-pH 7.0 | 10 millimolar |

Figure 1c shows the effect that a buffered medium can have on color formation. The reaction was run in Bausch & Lomb Sensitive Eyes Saline, Lot #GB506, with component concentrations listed below:

| Concentration of Reagents for Figure 1c | |
|---|---|
| Component | Concentration |
| peroxidase | 0.05 mg/ml |
| peroxide | 100 micromolar |
| sodium iodide | 0.15 mg/ml |

As compared to Figure 1, the intensity of color is meaningfully decreased: maximum optical density 0.145 as compared to 0.55. Additionally, the color decreases significantly over a twelve-minute time period to 0.073 optical density. That is, a level of temporal color control is effected.

### EXAMPLE 2

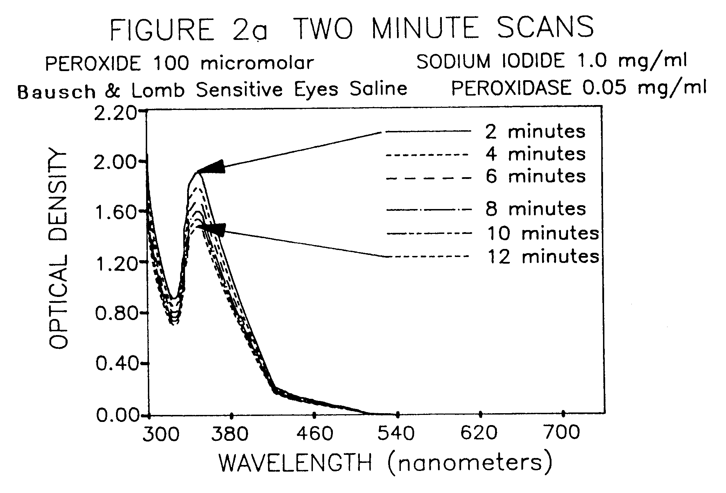

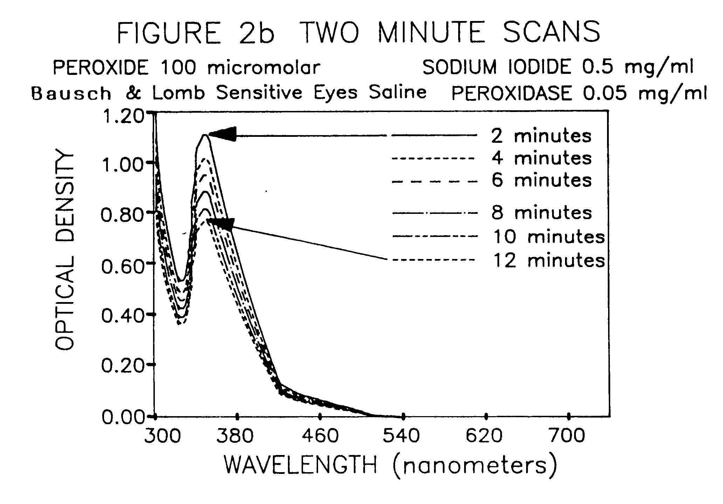

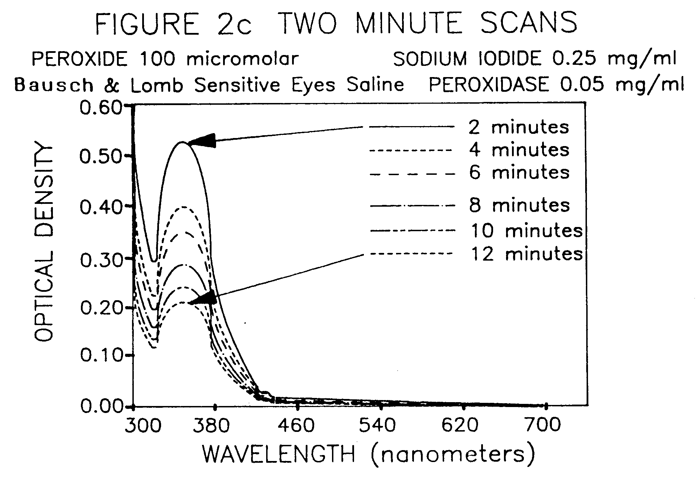

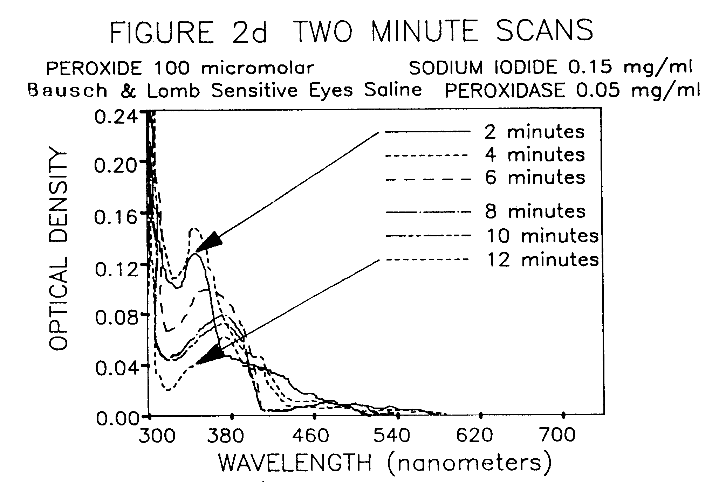

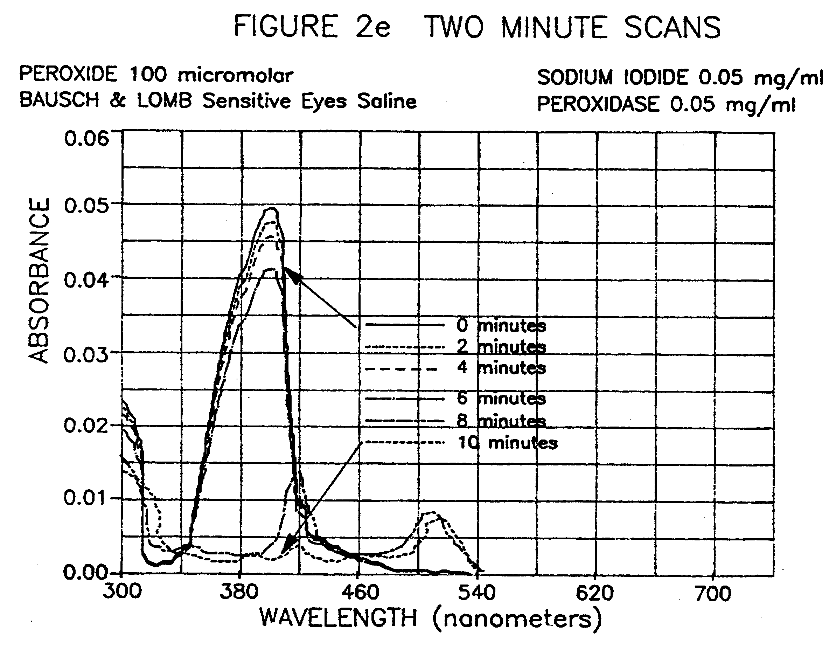

Figures 2 and 2e demonstrate one way in which the color can be caused to increase and then decrease to a noncolored condition. The conditions used for this measurement were as shown below:

| Component | Concentration |
|---|---|
| peroxidase | 0.05 mg/ml |
| peroxide | 100 micromolar |
| sodium phosphate-pH 7.0 | 10 millimolar |

| Concentration of Reagents for Figures 2a-2e | | | | | | |
|---|---|---|---|---|---|---|
| | Figure Number | | | | | |
| | 2 | 2a | 2b | 2c | 2d | 2e |
| mg/ml of iodide | 1.5 | 1.0 | 0.5 | 0.25 | 0.15 | 0.15 |

As the concentration of iodide is decreased, three things occur: (1) the intensity of the maximum absorbence decreases from 2.085 O.D. in Figure 2 to 0.043 in Figure 2e, (2) the absorbence maximum shifts from 355 nm in Figure 2 to 395 nm in Figure 2e, and (3) the percent decrease over a ten-minute period of the maximum absorbence in Figure 2 is 16 percent, as compared to 80 percent in Figure 2e. This allows the formulation of components so that the resulting solution appears colored to the user during the disinfection cycle, and then turns colorless after the period of disinfection. Figure 2f shows the maximum color formed at each of the iodide concentrations above. It is clear that the amount of color formed can be controlled by adding defined amounts of iodide for a given peroxide concentration and a given environment.

### EXAMPLE 3

Simple organic acids and salts were included in the reaction mixture to determine their effect on both the temporal and quantitative aspects of color formation. The reference reaction against which all reactions were compared contained the following components.

| Component | Concentration |
|---|---|
| peroxidase | 0.05 mg/ml |
| peroxide | 100 micromolar |
| sodium iodide | 0.15 mg/ml |
| sodium phosphate-pH 7.0 | 10 millimolar |

The UV-VIS spectrum was scanned from 200 nm to 450 nm six times during a ten-minute period. Other components were added to the reference reaction to determine their effect upon the amount and time course of color increase which was centered at 350 nm. Lactic acid, malonic acid, citric acid, and quiacol to concentrations of 0.1 percent by weight, did not act to reduce the intensity of color increase, nor to increase the rate of disappearance of color. Concentrations of sodium chloride up to 0.20 molar acted to reduce the intensity of color, but not the time course of color formation. In fact, 10 millimolar borate, pH 7.0 in combination with 0.15 molar sodium chloride and 0.01 percent sorbic acid eliminated color from the reference reaction.

## Claims

1. A method for controlling the formation of colour in a peroxide disinfecting system including, in combination, a peroxidase, peroxide and an iodide salt formulated in a desired ratio to cause disinfection within an aqueous medium, which comprises adding to said combination a decolorizer selected from sorbic acid, boric acid, thiol group containing compounds, sodium chloride and supplemental peroxide.

2. The method for controlling the formation of colour in a peroxide disinfecting system including, in combination, a peroxidase, peroxide and an iodide salt formulated in a desired ratio to cause disinfection within an aqueous medium, which comprises introducing a neutralizing agent, selected from pyruvic acid, copper ions, platinum and catalase, into said aqueous medium for interrupting the reaction between the aforesaid components of the disinfecting system upon reaching a minimum acceptable colour threshold level.

3. The method for controlling the formation of colour in a peroxide disinfecting system including, in combination, a peroxidase, peroxide and an iodide salt formulated in a desired ratio to cause disinfection within an aqueous medium, which comprises limiting the level of said iodide salt to a concentration corresponding to a minimum acceptable colour threshold level and adding tyrosine and/or phenol as second donor compound to cause additional disinfection.

4. A disinfection composition, comprising a peroxidase, a peroxide, an iodide salt, and a decolorizer selected from sorbic acid, thiol group containing compounds, boric acid and sodium chloride.

5. A disinfection composition comprising a peroxidase, a peroxide, an iodide salt and a neutralizing agent selected from pyruvic acid, copper ions, platinum and catalase to neuralize the disinfecting reaction after a preselected time period has elapsed.

6. A composition as defined in claims 4 and 5, wherein any two of the components iodide salt, peroxidase and peroxide are contained separately from the third.

## Patentansprüche

1. Verfahren zur Kontrolle der Farbbildung in einem desinfizierenden Peroxid-System, welches in Kombination eine Peroxidase, ein Peroxid und ein Iodsalz enthält, die in einem gewünschten Verhältnis formuliert sind, um eine Desinfizierung in einem wäßrigen Medium zu bewirken, wobei man zu der Kombination ein Entfärbungsmittel gibt, das ausgewählt ist unter Sorbinsäure, Borsäure, Thiolgruppen enthaltenden Verbindungen, Natriumchlorid und zusätzlichem Peroxid.

2. Verfahren zur Kontrolle der Farbbildung in einem desinfizierenden Peroxid-System, welches in Kombination eine Peroxidase, ein Peroxid und ein Iodsalz enthält, die in einem gewünschten Verhältnis formuliert sind, um eine Desinfizierung in einem wäßrigen Medium zu bewirken, wobei man ein neutralisierendes Mittel, das ausgewählt ist unter Brenztraubensäure, Kupferionen, Platin und Katalase, in das wäßrige Medium einführt, um die Reaktion zwischen den oben erwähnten Komponenten des desinfizierenden Systems zu unterbrechen, sobald ein akzeptabler, unterer Farbgrenzwert erreicht ist.

3. Verfahren zur Kontrolle der Farbbildung in einem desinfizierenden Peroxid-System, welches in Kombination eine Peroxidase, ein Peroxid und ein Iodsalz enthält, die in einem gewünschten Verhältnis formuliert sind, um eine Desinfizierung in einem wäßrigen Medium zu bewirken, wobei man den Gehalt an Iodsalz auf eine Konzentration begrenzt, die einem akzeptablen, unteren Farbgrenzwert entspricht, und Tyrosin und/oder Phenol als zweiten Donator zugibt, um eine weitere Desinfizierung zu bewirken.

4. Desinfizierende Zusammensetzung, umfassend eine Peroxidase, ein Peroxid, ein Iodsalz und ein Entfärbungsmittel, das ausgewählt ist unter Sorbinsäure, Thiolgruppen enthaltenden Verbindungen, Borsäure und Natriumchlorid.

5. Desinfizierende Zusammensetzung, umfassend eine Peroxidase, ein Peroxid, ein Iodsalz und ein neutralisierendes Mittel, das ausgewählt ist unter Brenztraubensäure, Kupferionen, Platin und Katalase und das zur Neutralisierung der desinfizierenden Reaktion nach Ablauf eines vorbestimmten Zeitraums dient.

6. Zusammensetzung nach den Ansprüchen 4 und 5, worin jeweils zwei der Komponenten Iodsalz, Peroxidase und Peroxid getrennt von der dritten enthalten sind.

## Revendications

1. Méthode pour contrôler la formation de couleur dans un système de désinfection à base de peroxyde comprenant, en combinaison, une peroxydase, un peroxyde et un sel d'iodure formulés dans des proportions souhaitées pour provoquer une désinfection dans un fluide aqueux, laquelle comprend l'addition à cette combinaison d'un décolorant choisi parmi l'acide sorbique, l'acide borique, les composés contenant un groupe thiol, le chlorure de sodium et une quantité supplémentaire de peroxyde.

2. Méthode pour contrôler la formation de couleur dans un système de désinfection à base de peroxyde comprenant, en combinaison, une peroxydase, un peroxyde et un sel d'iodure formulés dans des proportions souhaitées pour provoquer une désinfection dans un fluide aqueux, laquelle comprend l'introduction d'un agent neutralisant, choisi parmi l'acide pyruvique, les ions de cuivre, le platine et la catalase, dans ledit fluide aqueux, pour interrompre la réaction entre les composants précités du système de désinfection lorsqu'un niveau de seuil de couleur minimum acceptable est atteint.

3. Méthode pour contrôler la formation de couleur dans un système de désinfection à base de peroxyde comprenant, en combinaison, une peroxydase, un peroxyde et un sel d'iodure formulés dans des proportions souhaitées pour provoquer une désinfection dans un fluide aqueux, laquelle comprend la limitation du niveau dudit sel d'iodure à une concentration correspondant à un niveau de seuil de couleur minimum acceptable ainsi que l'addition de tyrosine et/ou de phénol comme second composé donneur, pour provoquer une désinfection supplémentaire.

4. Composition de désinfection, comprenant une peroxydase, un peroxyde, un sel d'iodure et un décolorant choisi parmi l'acide sorbique, les composés contenant un groupe thiol, l'acide borique et le chlorure de sodium.

5. Composition de désinfection, comprenant une peroxydase, un peroxyde, un sel d'iodure et un agent neutralisant choisi parmi l'acide pyruvique, les ions de cuivre, le platine et la catalase, pour neutraliser la réaction de désinfection après écoulement d'un laps de temps présélectionné.

6. Composition selon les revendications 4 et 5, dans laquelle deux quelconques des composants sel d'iodure, peroxydase et peroxyde sont contenus séparément du troisième.
